# EUROPEAN PATENT APPLICATION

(11) **EP 2 692 846 A2**
(43) Date of publication of application: **05.02.2014**
(21) Application number: 13178324.3
(22) Date of filing: 29.07.2013
(51) Int. Cl.: C12G 1/02, G01N 33/14

(54) **Improved fermenter**

(30) Priority: 30.07.2012 IT TV20120149
(71) Applicant: Noform S.r.l., 30020 Meolo (VE) (IT)
(72) Inventor: Crosato, Remo, 30020 Meolo (VE) (IT)
(74) Representative: Citron, Massimiliano

(57) **Abstract**

To improve the management and surveillance of a fermentation system (MC) or fermenter (10; 70) comprising a tank (10; 70) for containing material (14) to be fermented, in which the material can pass through successive fermentation phases, it is equipped with an indicator (50) which during each current phase of said phases is adapted to emit automatically a recognizable signal having a configuration related to the current phase.

## Description

The invention relates to an improved fermentation system, in particular for a winemaker for must herewith chosen as an example.

In tanks for winemaking, and wineries in general, the processes of winemaking are communicated or indicated to the operator in a very problematic way.

In the best case a tank, even if possibly equipped of a detection system of the fermentative stage inside it, sends relevant information to an electronic control panel or to a PLC connected next to the machine.

See e.g. U.S. 2003 097 937. The fermenter has a touch-screen that can show some process variables (temperature and level of the crushed grapes, oxygen concentration), and graphic pages, which the operator can select on the display, related to various processing stages. Each page summarizes and displays only the essential controls for each stage. In the event that an abnormality or accident to the fermenter occurs, an audible or visible alarm is emitted (siren or flashing lights).

The information presented on the display relate to the mechanical state of the fermenter, while the material into fermentation is monitored through the inspection of the process variables.

It is clear that the interaction man-tank/winemaker can improve, especially to facilitate or increase the inspection speed of the fermenters from a distance, or at a glance.

Making available an improved fermenter or system, in order to resolve one of these problems, is the main object of what is defined in claim 1, wherein it is proposed a system of fermentation or fermenter comprising
- a tank for containing material to be processed according to successive phases of fermentation,
   characterized by comprising
- an indicator that during each current phase of said phases is adapted (in use) to emit automatically (i.e. independently and without manual intervention) a signal recognizable (by a person) having configuration related (preferably uniquely) to the current phase.

The configurations of the signal express altogether, and let it be understood, the fermentation phase of the material and/or the mechanical state of the fermenter, thereby improving its management and surveillance. Unlike U.S. 2003 097 937, wherein the fermentation stage of the material is derived from many individual presentations of data and one must navigate through different pages (with an operator present!), the system can constantly detect such a stage (by appropriate sensors and/or by elaborating various parameters) and automatically generate the corresponding indicative signal.

Preferably the configuration or characteristic of the signal is variable as a univocal function of said phases and/or the stage of fermentation.

The signal is preferably continuous, and not e.g. emitted at intervals, to maximize the informative effectiveness.

A very immediate type of signalling is a light and/or acoustic signal, especially where its frequency (pitch or timbre of sound or color of the emitted light) is changed.

E.g. a light emitter can be applied to a tank (e.g. a lamp, a beacon or a multicolour-LED lamp), preferably connected to a control PLC of the relative winemaker and/or to an overall control PLC of many fermenters, and assisted by a detection system of the fermentative stage in each tank.

In this way a system of dynamic display of the stage reached by the fermentation processes is achieved.

A light indicator e.g. can emit a wide or predetermined color spectrum; or predefined color scales or hues, ranging from green to red, passing through the cyans, blues and pinks (but other colors are possible), or chromatic tones that vary with continuity from one color to another.

In any case, the colors and/ or color shades are related to the particular fermentation phase and/or to the particular stage of winemaking (e.g. the blue range when the tank is saturated by inert gases, the range of the green when in the fermenter maceration is carried out, the range of the red when alcoholic fermentation takes place in the winemaker, etc..).

In particular, the system (or in general a fermenter) can comprise means for programming
- the variability of the signal as a function of the fermentation stage of the material or of said phases, and/or in particular
- the association between the various configurations of the emitted signal by the indicator and said phases.

Such means, e.g. a PLC or computer, can e.g. allow, by using appropriate software or program, to
define the type of signal (e.g. frequency, duration, amplitude, signal configuration, etc.) that corresponds to one of said phases or to one particular phase of fermentation, and/or
- define how the signal is emitted (modulation, timing, duty-cycle, pauses, etc. ).

It is apparent that each processing phase can easily become recognizable by the operator.

In particular, the color and/or the color shades of said phases and/or their univocal association to the various fermentation phases can advantageously be pre-set or programmable/settable as desired by the user via said management means.

With the system claimed herein it is possible to build a winery wherein to each tank or winemaker there is related a lighting system that immediately indicates the situation not only of a single fermenter, but also globally of the winemaking/fermentation processes taking place in the winery.

The indicator can be applied e.g. at any place on the outer surface of the winemaker or tank, e.g. on the roof, on the bottom, on the side, and if the indicator is luminous it must be in a visible position.

The system allows a new approach to both the design of the wineries and the work inside them, since by the system what is going on inside the tanks or the winemakers becomes immediately comprehensible, even to those who are busy with other work and/or far away. An operator away from the centralized control panel or from that placed on board of the winemakers can however monitor the evolution of the processes.

The wineries from mere places of production have become also essential legs of wine tourism itineraries. By the described system even the inexperienced visitor can understand in a clear, immediate and unequivocal manner what is happening in the winery.

It is clear that the system allows a winery-attendant or an oenologist to quickly check all the processes that are taking place in each tank/winemaker, and therefore, ultimately, to be able to intervene more quickly and produce a better wine.

Several tanks, e.g. each of which is equipped with its own control panel, possibly with a PLC, can be connected to a remote PLC or computing unit that controls the indicators thereof. One can even provide a centralized PLC or control panel to manage all the detection systems of the fermentation/winemaking stages and/or the indicators that translate into a visible signal these stages. Such a system is defined in claim 8.

In the system the indicator can be adapted to emit a beam of waves (sound or light) facing the outer surface of the tank, which usually is metallic and reflective, so that the visual range of the signal is increased. With the same advantage the beam can be facing a wall of the environment that contains the tank.

It is also proposed a method to indicate the fermentation stage of a fermenting material contained within a tank, see Claim 10.

Through the emission of a signal that has different configurations related to said phases the immediacy of the perception of the processes in place improves. The peculiarities described for the system are to be considered integrated and integratable into the method, and will not be repeated here.

A software can control the operation of said indicator and/or of the overall system, and also can allow the execution of the method.

The advantages of the invention will be more apparent from the following description of a preferred embodiment, making reference to the attached drawing in which
- Figure 1 shows a front view of an improved fermenter,
- Figure 2 shows a front view of a variant of improved fermenter,
- Figure 3 shows a front view of a variant of improved fermenter.

In the description and in the various figures the same references indicate equal parts.

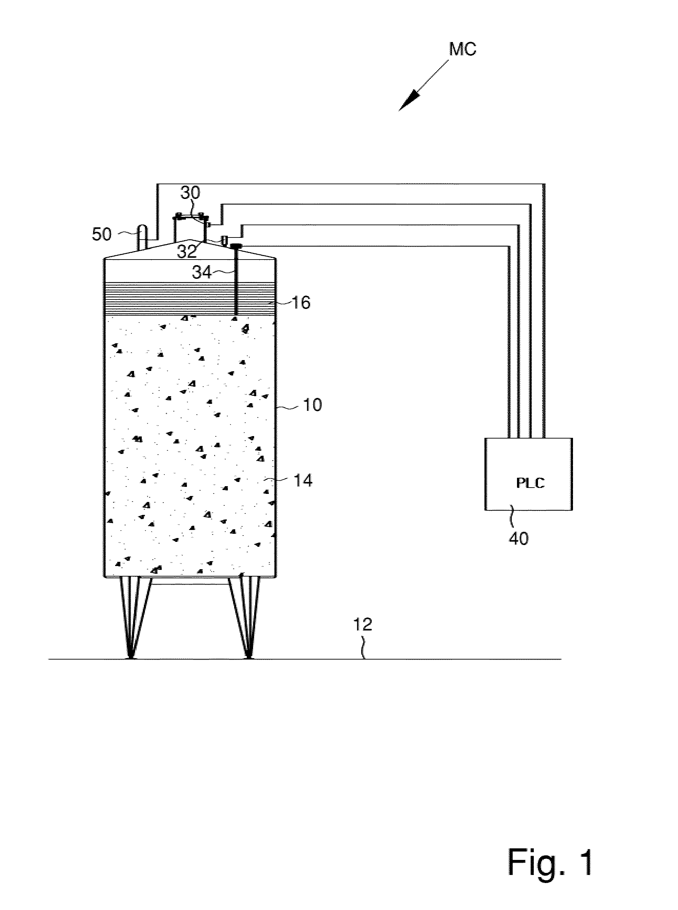

A fermentation system MC (Fig. 1) comprises, for example, a winemaker having a fermentation tank 10 placed on a support floor 12. The tank 10 contains must 14 that fermenting has generated a marc cap 16.

The tank 10 is equipped with sensors, such as a pressure transducer 30 (in the vicinity of a top hatch), a level probe 34 and a valve 32 for regulating gas pressure.

The sensors 30, 32, 34 are connected to a processing unit 40, e.g. a PLC, which controls a luminous device 50 placed on the roof of the tank 10.

The device 50 is capable of emitting and spreading light of different colors (e.g. a multicolor LED).

The unit 40 reads the sensors 30, 32, 34 and manages all the mechanical and operational functions of the winemaker, like e.g. the management of the valves, the injection of gas, the loading/unloading of the must 14. Preferably the unit 40 shows information to an operator on a touch-screen.

Via the sensors 30, 32, 34, or others not shown, the unit 40 is able to determine the fermentation stage of the must 14.

Depending on the type of programming that has been set, the unit 40 controls the device 50, in particular the color emitted. Each color is made to be corresponding to a fermentation phase of the must 14 and/or a processing phase of the winemaker.

E.g. below is shown a table of correspondence:
the tank 10 is empty and is saturated with inert gas → blue color;
the tank 10 is filled with crushed vegetable material and is into maceration phase ←→ green color;
the tank 10 is into the process of alcoholic fermentation, with the fermentation just started ←→ pink color;
the tank 10 is into advanced alcoholic fermentation ←→ red color.

By the unit 40 one can associate at will a color and/or cromatic shades of color to the various stages of wine-making or fermentation.

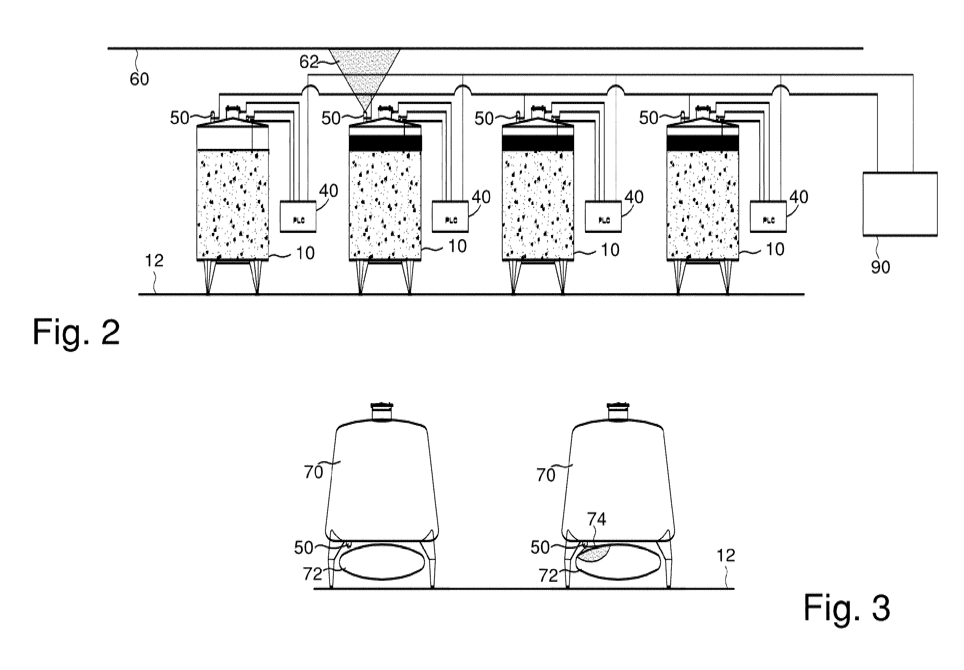

In general, the device 50 can have a 360° emission or a directional emission, like a beam (e.g. laser) or a light cone (indicated by 62 in figure 2). For this purpose focusing devices, mirrors, reflective surfaces and/or reflecting means can be used.

In particular, the light emission can be directed towards a surface, preferably reflective, to amplify the visual effect.

In fig. 2 some devices 50 are oriented or adapted to send the colored light, see beam 62, toward a ceiling 60, on which there is formed a colored stain being very visible from afar.

In fig. 3 is shown a tank 70, e.g. frustoconical in shape, having under its bottom an auxiliary tank 72 and a device 50 mounted to send the emitted light toward the tank 72. The tank 72 is preferably metallic, reflective (e.g. because of it being built of steel) and preferably with curved walls, so that the effect of reflection is increased.

In fig. 3 on the left there is shown the empty and inactive tank 70, and the device 50 does not emit light.

In fig. 3 on the right the tank 70 is filled with crushed material and it is into processing phase. The light beam 74, which invests the tank 72, is visible.

Fig 2 also represents the case of multiple tanks 10, each provided with a unit 40, in turn connected to a centralized processing unit 90 which controls the lighting systems 50. The control panel 90 can receive from the units 40 the data relative to the winemakers and/or to the fermentation/winemaking stages, or can manage all systems and detection means such as the sensors 30, 32, 34 of each tank 10.

## Claims

1. Fermentation system (MC) or fermenter (10; 70) comprising
- a tank (10; 70) for containing material (14) to be fermented, in which the material can pass through successive fermentation phases, **characterized by** comprising
- an indicator (50) which during each current phase of said phases is adapted to emit automatically a recognizable signal having a configuration related to the current phase.

2. System according to claim 1, wherein the signal has configurations of different frequency related to each of said phases.

3. System according to any of the preceding claims, wherein the indicator is mounted on the outer surface of the tank.

4. System according to any of the preceding claims, wherein the signal is luminous and/or acoustic.

5. System according to any of the preceding claims, wherein the indicator is adapted to emit a beam of waves (74) directed toward the outer surface of the tank.

6. System according to any of the preceding claims, wherein the indicator is adapted to emit a beam of waves (62) directed toward a wall of the environment that contains the tank.

7. System according to any of the preceding claims, comprising
means (40, 90) for programming the association between some signal configurations emitted by the indicator and said phases.

8. Apparatus comprising two or more systems according to one of claims 1 to 7, comprising a processing unit adapted to remotely control the indicators of such systems.

9. Apparatus according to claim 8, wherein the processing units is adapted (i) to detect the fermentation stage of the material in each tank of said systems and (ii) to control, as a function of such stage, the signal emitted by each indicator.

10. Method for indicating the fermentation stage of a material into fermentation contained within a tank (40), said material being able to pass through various processing phases,
**characterized by** automatically emitting during each of said phases a recognizable signal having configuration related to the current phase.
